(19) Europäisches Patentamt / European Patent Office / Office européen des brevets

(11) **EP 4 691 486 A1**

(12) **EUROPEAN PATENT APPLICATION**
published in accordance with Art. 153(4) EPC

(43) Date of publication:
**11.02.2026 Bulletin 2026/07**

(21) Application number: **24781312.4**

(22) Date of filing: **29.03.2024**

(51) International Patent Classification (IPC):
*A61K 39/39* (2006.01)    *A61K 39/09* (2006.01)
*A61P 31/04* (2006.01)    *A61K 39/00* (2006.01)

(52) Cooperative Patent Classification (CPC):
**A61K 39/00; A61K 39/09; A61K 39/39; A61P 31/04**

(86) International application number:
**PCT/KR2024/004079**

(87) International publication number:
**WO 2024/205322 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 KR 20230043089**

(71) Applicant: **SK Bioscience Co., Ltd.**
**Gyeonggi-do 13494 (KR)**

(72) Inventors:
• **LEE, Jeong Min**
**Seongnam-si, Gyeonggi-do 13494 (KR)**
• **KIM, Min Ji**
**Seongnam-si, Gyeonggi-do 13494 (KR)**
• **SHIN, Jin Hwan**
**Seongnam-si, Gyeonggi-do 13494 (KR)**

(74) Representative: **Weickmann & Weickmann PartmbB**
**Postfach 860 820**
**81635 München (DE)**

(54) **METHOD FOR PREPARING ALUMINUM-BASED ADJUVANT HAVING ENHANCED EFFICACY**

(57) The present disclosure provides: a method of preparing an aluminum-based adjuvant having enhanced efficacy, the method comprising a plurality of processes of performing an autoclave treatment process; an aluminum-based adjuvant prepared by the method; an immunogenic composition including the aluminum-based adjuvant; and a kit including the immunogenic composition. The aluminum-based adjuvant prepared by the method may be used to improve the safety and effectiveness of conventional vaccine formulations, including polysaccharide vaccines and polysaccharide-protein conjugate vaccines.

FIG. 1

EP 4 691 486 A1

## Description

Technical Field

The present disclosure relates to a method of preparing an aluminum-based adjuvant having enhanced efficacy. This application claims priority from Korean Patent Application No. 10-2023-0043089, filed on March 31, 2023, the disclosure of which is incorporated herein by reference.

Background Art

Vaccines with preventive or protective effects against infectious diseases have been developed in various forms since the introduction of the live smallpox vaccine. In the early days of vaccine development, vaccines were developed in the form of live vaccines or inactivated vaccines; however, problems arose such as systemic or local side effects, and the possibility of causing other diseases. Accordingly, many vaccines have been developed in the form of subunit vaccines that use polysaccharides or proteins as antigens. However, such subunit vaccines have lower immunogenicity than live or inactivated vaccines, and therefore substances having immune-enhancing activity, i.e., adjuvants, have been mixed with the target antigen for use. The adjuvant is a substance added to a vaccine formulation to increase immunogenicity and plays a role in rapidly and strongly inducing an immune response to the antigen, even when the amount of antigen is small, and in maintaining the immune response for a long time. Known adjuvants or their components to date include aluminum salts, saponins, Toll-like receptor (TLR) agonists, and oil-in-emulsion forms such as MF59, AS03, and AF03.

Meanwhile, the most widely used adjuvant in most commercialized vaccines is an aluminum adjuvant, or so-called "alum," which is a precipitated aluminum salt. The aluminum adjuvant is usually aluminum hydroxide or aluminum phosphate, but amorphous aluminum hydroxyphosphate sulfate is also used. It is known that aluminum adjuvants, due to the particle properties of the aluminum salt, act as a depot for the vaccine antigen to increase the local antigen concentration, enhance the antigen uptake ability of antigen-presenting cells (APCs), and enhance humoral immune responses such as antibody production. However, despite such effects, aluminum adjuvants have only minimal effects in inducing cellular immune responses, such as antibody-dependent cell cytotoxicity (ADCC) or cytotoxic T lymphocyte (CTL) responses, and thus there are cases where the protective effect from vaccination is insufficient. Additionally, aluminum salts, when used in subcutaneous or intradermal injection formulations, have a high likelihood of forming granulomas at the injection site, and therefore are mainly applied to intramuscular injection vaccine formulations.

Against this technical background, although various attempts to enhance the efficacy of aluminum adjuvants as a technique for improving the effectiveness and stability of vaccine formulations have been reported (see Korean Patent No. 10-1540920), the results are still insufficient.

Disclosure

Technical Problem

An aspect provides a method of preparing an aluminum-based adjuvant including a plurality of processes of an autoclave treatment in a solution containing aluminum salt particles.

Another aspect provides an aluminum-based adjuvant prepared by the method.

Another aspect provides an immunogenic composition including the aluminum-based adjuvant.

Another aspect provides a kit including the immunogenic composition.

Another aspect provides a prefilled syringe filled with the immunogenic composition.

Other objects and advantages of the present disclosure will become more apparent from the following detailed description together with the accompanying claims and drawings. Descriptions not provided in the present specification may be sufficiently recognized and inferred by those skilled in the art or in similar technical fields and are therefore omitted.

Technical Solution

An aspect provides a method of preparing an aluminum-based adjuvant, including a plurality of processes of an autoclave treatment while sequentially increasing temperature conditions in a solution containing aluminum salt particles.

The term "adjuvant" as used herein refers to a formulation or substance that is used together with a target antigen to increase the clinical efficacy of a vaccine formulation, and it functions during and after administration of the vaccine formulation to an individual or mammal. The adjuvant may, for example, be a formulation or substance that enhances the magnitude, duration, and/or specificity of an immune response stimulated by the target antigen, or induces the immune response in a desired direction. Among the adjuvants, aluminum-based adjuvants are most widely used in that they use an insoluble salt to provide excellent antigen adsorption and high stability in a vaccine formulation; however, due to relatively low immune-enhancing efficiency and weak cellular immune response induction ability, additional technological development is needed. Under this technical background, the present disclosure has confirmed that, by performing an autoclave treatment under certain conditions on a solution containing aluminum salt particles, it is possible to enhance the immune response compared to existing vaccine formulations containing aluminum-based adjuvant.

A detailed description of the method of preparing an aluminum-based adjuvant is as follows.

The autoclave treatment may be performed in a manner including: a first process of applying steam at a condition of 101 °C to 103 °C for 2 minutes to 4 minutes to a solution containing aluminum salt particles; a second process of applying steam at a condition of 104 °C to 106 °C for 20 minutes to 30 minutes to the solution having undergone the first process; and a third process of applying steam at a condition of 110 °C to 126 °C for 220 minutes to 260 minutes to the solution having undergone the second process.

The term "autoclave treatment" as used herein refers to a series of processes that provide high-temperature, high-pressure steam to a subject placed in a sealed reaction vessel, and may include providing a temperature condition of 100 °C or higher and a pressure condition of 1 atmosphere (15 psi) or higher to the subject. In the art, the autoclave treatment is used as one of the sterilization methods; however, in the present disclosure, the autoclave treatment has technical significance in that it may not only sterilize the aluminum-based adjuvant solution but also significantly enhance the immunogenicity of the target antigen.

In an embodiment, the autoclave treatment may be performed in multiple processes while sequentially increasing the temperature condition, and the processes may, for example, be configured by setting the initial temperature to 102 °C and the final temperature to 118 °C and then further setting at least one or more temperature conditions between 102 °C and 118 °C, thereby constituting multiple processes. The autoclave treatment time for each process may be independently the same or different. Here, the initial temperature may, for example, be any one of 101 °C to 103 °C, and the final temperature may be any one of 110 °C to 126°C.

In an embodiment, the autoclave treatment may, for example, include: a first process of applying steam at a condition of 101 °C to 103°C for 2 minutes to 4 minutes to a solution containing aluminum phosphate particles; a second process of applying steam at a condition of 104 °C to 106 °C for 20 minutes to 30 minutes to the solution having undergone the first process; and a third process of applying steam at a condition of 110 °C to 126 °C for 220 minutes to 260 minutes to the solution having undergone the second process. In the autoclave treatment, for example, the first process may include applying steam at a condition of 101 °C to 103 °C, 101 °C to 102 °C, 102 °C to 103 °C, or 102$\pm$0.1 °C for 2 minutes to 4 minutes, 2 minutes to 3 minutes, or 3 minutes to 4 minutes; the second process may include applying steam at a condition of 104 °C to 106 °C, 104 °C to 105 °C, 105 °C to 106 °C, or 105$\pm$0.1 °C for 20 minutes to 30 minutes, 20 minutes to 28 minutes, 20 minutes to 26 minutes, 20 minutes to 24 minutes, 20 minutes to 22 minutes, 25 minutes to 30 minutes, 25 minutes to 28 minutes, or 25 minutes to 26 minutes; and the third process may include applying steam at a condition of 110 °C to 126 °C, 110 °C to 123 °C, 110°C to 120 °C, 110 °C to 117 °C, 110 °C to 114 °C, 110°C to 111 °C, 113 °C to 126 °C, 113 °C to 123 °C, 113 °C to 120 °C, 113 °C to 117 °C, 113 °C to 114 °C, 116 °C to 126 °C, 116 °C to 123 °C, 116 °C to 120 °C, 116 °C to 117 °C, or 118$\pm$0.1 °C for 220 minutes to 260 minutes, 220 minutes to 250 minutes, 220 minutes to 240 minutes, 220 minutes to 230 minutes, 230 minutes to 260 minutes, 230 minutes to 250 minutes, or 230 minutes to 240 minutes. If the temperature and processing time conditions are outside these ranges, the stability of the aluminum salt particles may decrease, the aluminum salt particles may break, and the level of immune response induction by the mixed target antigen may be weak. In particular, if the starting temperature of the autoclave treatment is higher than the above-mentioned conditions or if the processing time of each process exceeds the above-mentioned conditions, aggregation of the aluminum salt particles may occur, leading to a decrease in uniformity. Furthermore, if the processing time of the third process is shorter than the above-mentioned conditions, there is a concern that the efficacy of the adjuvant may be reduced due to the influence of contamination factors from the external environment; and if the processing time of the third process is longer than the above-mentioned conditions, the concentration and viscosity of the aluminum salt may increase, making the results of the manufacturing process inconsistent.

In addition, in the autoclave treatment, the pressure condition inside the sealed reaction vessel (for example, sterilizer chamber) in which the autoclave is performed may be in the range of 0 bar to 1.3 bar. For example, the autoclave condition of the first process may be set to 0 bar, and it may be gradually increased so that, at the start of the third process of autoclave treatment, it reaches 1.3 bar, for example, 1.3$\pm$0.1 bar, and during the autoclave treatment of the third process, 1.3$\pm$0.1 bar may be maintained for a certain period of time.

In an embodiment, the method may further include a process of performing pre-vacuum on the sealed reaction chamber in which the autoclave is performed before the autoclave treatment described above. The pre-vacuum process may be for supplying saturated steam into the chamber in the subsequent autoclave process by applying a vacuum/-pressure pulse to the reaction chamber. In an embodiment, the pre-vacuum process may be performed under conditions where the bypass pipe connected to the reaction chamber is closed, and under the condition of 0 bar and 1 counter setting. The pre-vacuum process may be performed under the condition where the bypass pipe is closed, so that steam does not escape, thereby efficiently increasing the temperature inside the reaction chamber. In addition, the pre-vacuum process may be performed under the condition where 0 bar is maintained at the 0 bar and 1 counter setting, so that the autoclave may be performed under stable conditions without leakage of the solution containing the aluminum salt particles.

In an embodiment, the method may further include a process of cooling down after the above-described autoclave treatment. The cooling down may be performed, for example, at room temperature conditions, for 20 minutes to 40 minutes, 20 minutes to 35 minutes, 20 minutes to 30 minutes, 20 minutes to 25 minutes, 30 minutes to 40 minutes, 30 minutes to 35 minutes, or 35 minutes to 40 minutes.

In an embodiment, the solution containing aluminum salt particles may be any one of an aluminum phosphate solution, an aluminum sulfate solution, an aluminum hydroxide solution, a potassium aluminum sulfate solution, and an aluminum hydroxyphosphate solution, and depending on the selection of the solution containing aluminum salt particles, the aluminum-based adjuvant may be any one of an aluminum phosphate adjuvant, an aluminum sulfate adjuvant, an aluminum hydroxide adjuvant, a potassium aluminum sulfate adjuvant, and an aluminum hydroxyphosphate adjuvant. In addition, in an embodiment, the aluminum salt particles may be sterilized, and for example, may be sterilized at least 121 °C for at least 30 minutes.

In an embodiment, the aluminum-based adjuvant may have one or more of the following effects compared to an aluminum adjuvant produced without performing autoclave or by performing autoclave under temperature/processing time conditions different from those of the embodiment:

(a) enhanced loading of the target antigen;
(b) enhanced adsorption rate with the target antigen; and
(c) enhanced immunogenicity of the target antigen.

As used herein, the term "target antigen" refers to a substance that may initiate and mediate an immune response, and is an antigen that stimulates or enhances an immune response; the terms "immunogen" and "vaccine antigen" may be used interchangeably. The immune response stimulated by the target antigen may be either humoral or cellular, or both, and accordingly, the target antigen may be a substance that may be bound by an antibody molecule or by a T cell receptor. The target antigen may, for example, be a protein, carbohydrate, polysaccharide, oligosaccharide, lipid, phospholipid, metabolite, hormone, nucleic acid and other molecules, and fragments thereof, mixtures thereof, or conjugates thereof, and may, for example, be any one of a protein antigen, recombinant protein antigen, polysaccharide antigen, and polysaccharide-protein conjugate antigen.

In an embodiment, the target antigen may include or be a part of a component larger than a single molecule, such as all or part of a cell, a bacterium, a virus, or other microorganisms. For example, bacterial antigens may include proteins, polysaccharides, and other molecules derived from the outer surface, interior of the cell, flagella, or other components. For example, the target antigen may be a pneumococcal polysaccharide antigen, Haemophilus influenzae type b antigen, hepatitis A antigen, hepatitis B antigen, human papillomavirus antigen, anthrax antigen, Escherichia coli (*E. coli*) antigen, rabies antigen, influenza antigen, or the like, and preferably, may be a pneumococcal polysaccharide-protein conjugate antigen. Here, the serotypes of the pneumococcal polysaccharide may be one or more selected from 1, 2, 3, 4, 5, 6A, 6B, 6C, 5D, 7A, 7C, 7F, 8, 9N, 9V, 10A, 10F, 11A, 11B, 12F, 13, 14, 15A, 15B, 15C, 15F, 16F, 17F, 18C, 19A, 19B, 19F, 20, 20A, 21, 22F, 23A, 23B, 23F, 24F, 25A, 31, 33F, 35A, 35B, 35D, 35F, 38, and 39, and more preferably, may be one or more selected from 1, 3, 4, 5, 6A, 6B, 7F, 8, 9N, 9V, 10A, 11A, 12F, 14, 15B, 18C, 19A, 19F, 22F, 23F, and 33F, but is not limited thereto.

Meanwhile, the method may non-restrictively apply technologies known in the art that are associated with the production of aluminum-based adjuvants. For example, the solution containing aluminum phosphate particles may be prepared through a series of processes including: a process of preparing an aluminum salt solution and/or a phosphate solution; a process of preparing an aluminum phosphate solution by mixing the aluminum salt solution and the phosphate solution; and a process of removing residual salts in the aluminum phosphate solution, and in each of the processes, the components, contents, and reaction conditions, etc. may be adopted and performed from known technologies.

According to the method of an aspect, the adjuvant prepared through the above-described series of processes could improve the antigen content in the vaccine formulation and the adsorption rate to the antigen, thereby enhancing the induction of an immune response by vaccination, and it was confirmed that it may be used to improve the efficacy of existing vaccine formulations including polysaccharide vaccines and polysaccharide-protein conjugate vaccines.

Specifically, in the present examples, by finely controlling the autoclave application temperature, an optimal $AlPO_4$ formulation that may be used in a vaccine composition was provided, and as an effect derived from a series of processes including three autoclave treatments under specific temperature/time conditions, it was confirmed that, unlike autoclave treatment processes under other temperature/time conditions, the adjuvant could be formulated without the formation of aluminum phosphate particles in the adjuvant while achieving a high antigen content level and antigen adsorption rate in the vaccine formulation. In addition, in the present examples, when the primarily sterilized $AlPO_4$ was subjected to autoclave treatment at specific temperature/time conditions according to an example, it was confirmed that uniformity was maintained not only at the lab scale but also at the commercial scale, making it easy to formulate, advantageous for ensuring the safety of finished pharmaceutical products, and that in addition to excellent properties in terms of appearance/formulation, a remarkable effect of enhanced immunogenicity was achieved even in actual animal tests.

Another aspect provides an aluminum-based adjuvant prepared by the method.

Any term or element mentioned in the aluminum-based adjuvant or the aluminum phosphate adjuvant below that is the same as that mentioned in the description of the method of preparing the aluminum-based adjuvant is as described above.

The aluminum-based adjuvant, as described above, may, compared to an aluminum adjuvant produced without performing autoclave or by performing autoclave under temperature/processing time conditions different from those of one embodiment, have one or more of: enhanced loading of the target antigen; enhanced adsorption rate with the target antigen; suppression of aggregation of the target antigen under room temperature conditions; and enhanced immunogenicity of the target antigen. Here, the adsorption with the target antigen may be referred to as "binding of antigen to adjuvant" or "binding between antigen and adjuvant." The adsorption with the target antigen may be calculated by removing the supernatant containing the target antigen that is not adsorbed to the adjuvant in the vaccine formulation using centrifugation, resuspending the precipitate containing the adjuvant and the adsorbed target antigen using a vehicle, and then calculating the ratio (%) of the target antigen content in the resuspended vaccine formulation to the target antigen content in the vaccine formulation. In an embodiment, at least 15 % or more of the target antigen present in the vaccine formulation may be adsorbed to the adjuvant, and for example, the ratio of the target antigen adsorbed to the adjuvant present in the vaccine formulation may be at least 20 % or more, at least 30 % or more, at least 40 % or more, or at least 50 % or more. In an embodiment, the target antigen may be a pneumococcal polysaccharide-protein conjugate antigen.

In an embodiment, the pH of the aluminum adjuvant may be 5.0 to 7.0, and the pH may, for example, be 5.2 to 7.0, 5.4 to 7.0, 5.6 to 7.0, 5.8 to 7.0, 6.0 to 7.0, 6.2 to 7.0, 6.4 to 7.0, 6.6 to 7.0, 6.8 to 7.0, 5.0 to 6.6, 5.2 to 6.6, 5.4 to 6.6, 5.6 to 6.6, 5.8 to 6.6, 6.0 to 6.6, 6.2 to 6.6, 6.4 to 6.6, 5.0 to 6.2, 5.2 to 6.2, 5.4 to 6.2, 5.6 to 6.2, 5.8 to 6.2, or 6.0 to 6.2.

Another aspect provides an immunogenic composition including: a polysaccharide-protein conjugate including a pneumococcal serotype polysaccharide antigen; and the aluminum-based adjuvant.

Any term or element mentioned in the immunogenic composition below that is the same as that mentioned in the description of the method of preparing the aluminum-based adjuvant, the aluminum-based adjuvant, and the aluminum phosphate adjuvant, is as described above.

As used herein, the term "immunogenic composition" refers to a substance that contains an active ingredient effective in inducing a certain degree of immunity in a subject against a specific pathogen or disease, and the terms "vaccine," "vaccine preparation," and "vaccine composition" may be used interchangeably. Additionally, the immunogenic composition may be a pharmaceutical composition that induces a decrease in the severity, duration, or other signs of symptoms associated with infection by a disease or pathogen. Therefore, the immunogenic composition may further include a pharmaceutically acceptable diluent, carrier, excipient, buffer, salt, surfactant, cryoprotectant, or the like.

In the immunogenic composition or vaccine formulation, the buffer is not particularly limited as long as it is known to be used in pharmaceutical compositions, particularly in vaccine formulations, and examples thereof include histidine, citrate, phosphate, succinate, or Hepes (4-(2-hydroxyethyl)-1-piperazineethanesulfonic acid). Additionally, the salt may be selected from the group consisting of magnesium chloride, potassium chloride, sodium chloride, borate chloride, and combinations thereof, and the surfactant may be selected from the group consisting of polysorbate 20, polysorbate 40, polysorbate 60, polysorbate 65, polysorbate 80, polysorbate 85, Triton N-101, Triton X-100, octoxynol 40, nonoxynol-9, triethanolamine, triethanolamine polypeptide oleate, polyoxyethylene-660 hydroxystearate (PEG-15, Solutol H 15), OTG (octylthioglucoside), OG (octylglucoside), NM (nonylmaltoside), LDAO (lauryldimethylamine oxide), DDM (dodecyl beta-D-maltoside), and poloxamer.

In the immunogenic composition or vaccine formulation, the pH of the vaccine formulation may be 5.0 to 7.0, and the pH may, for example, be 5.2 to 7.0, 5.4 to 7.0, 5.6 to 7.0, 5.8 to 7.0, 6.0 to 7.0, 6.2 to 7.0, 6.4 to 7.0, 6.6 to 7.0, 6.8 to 7.0, 5.0 to 6.6, 5.2 to 6.6, 5.4 to 6.6, 5.6 to 6.6, 5.8 to 6.6, 6.0 to 6.6, 6.2 to 6.6, 6.4 to 6.6, 5.0 to 6.2, 5.2 to 6.2, 5.4 to 6.2, 5.6 to 6.2, 5.8 to 6.2, or 6.0 to 6.2.

As used herein, the term "effective amount" refers to an amount of administration necessary to induce an antibody to a degree that may significantly reduce the probability or severity of infection by a specific pathogen or disease. The administration may include intramuscular, intraperitoneal, intradermal, or subcutaneous injection, or mucosal administration through the oral/gastrointestinal tract, respiratory tract, or genitourinary tract. The effective amount of the target antigen is selected in an amount that induces an immune response without significant side effects. Generally, the dose of the target antigen may be 0.1 μg to 100 μg, preferably 0.1 μg to 10 μg, more preferably 1 μg to 5 μg, but is not limited thereto. The optimal amount of a component in a specific vaccine formulation may be confirmed by standard studies including observation of an appropriate immune response in a subject. For example, the dose of a vaccine for humans may be determined by extrapolating from animal test results or may be determined empirically.

In an embodiment, the immunogenic composition or vaccine formulation may be a pneumococcal polysaccharide-protein conjugate, and the serotype of the pneumococcal polysaccharide may be one or more of 1, 2, 3, 4, 5, 6A, 6B, 6C, 5D, 7A, 7C, 7F, 8, 9N, 9V, 10A, 10F, 11A, 11B, 12F, 13, 14, 15A, 15B, 15C, 15F, 16F, 17F, 18C, 19A, 19B, 19F, 20, 20A, 21, 22F, 23A, 23B, 23F, 24F, 25A, 31, 33F, 35A, 35B, 35D, 35F, 38, and 39, and more preferably may be one or more selected from 1, 3, 4, 5, 6A, 6B, 7F, 8, 9N, 9V, 10A, 11A, 12F, 14, 15B, 18C, 19A, 19F, 22F, 23F, and 33F, but is not limited thereto. For example, the vaccine formulation may be a sterilized liquid formulation of a pneumococcal polysaccharide-protein conjugate antigen, each conjugated to $CRM_{197}$, and in this case, based on each 0.5 mL single dose, it may contain 2 μg or 4 μg of each polysaccharide; about 34 μg of $CRM_{197}$ carrier protein; and 0.5 mg of the aluminum-based adjuvant, but may be formulated to contain sodium chloride, succinate as a buffer, and any one of polysorbate 80 or polysorbate 20 as a surfactant. In an embodiment, the vaccine formulation may be formulated as a single 0.5 mL dose using a mixed carrier to contain about 1 μg to about 5 μg of capsular polysaccharide; about 1 μg to about 30 μg of TT; about 20 μg to about 85 μg of $CRM_{197}$; and optionally about 0.1 mg to about 0.5 mg of elemental aluminum adjuvant. Additionally, in an embodiment, the vaccine formulation may be formulated as a single 0.5 mL dose to contain about 2 μg to about 2.5 μg of each capsular polysaccharide except for up to 6 capsular polysaccharides selected from the group consisting of serotypes 1, 3, 4, 5, 6B, 9V, 19A, and 19F, and each of the selected up to 6 capsular polysaccharides may be present in an amount of about 4 μg to about 5 μg. The vaccine formulation may be formulated as a single 0.5 mL dose to contain about 2.2 μg of each capsular polysaccharide except for up to 6 capsular polysaccharides selected from the group consisting of serotypes 1, 3, 4, 5, 6B, 9V, 19A, and 19F, and each of the selected up to 6 capsular polysaccharides may be present in an amount of about 4.4 μg. As an example, the capsular polysaccharides present in an amount of about 4.4 μg may be serotypes 3, 4, 6B, 9V, 19A, and 19F, and the capsular polysaccharides present in an amount of about 2.2 μg may be serotypes 1, 5, 15B, 22F, 6A, 7F, 8, 9N, 10A, 11A, 12F, 14, 18C, 23F, and 33F, but are not limited thereto. The liquid formulation may be filled in a single-dose syringe without a preservative and, when shaken, may be produced as a homogeneous white suspension that may be administered intramuscularly immediately.

According to an embodiment, the immunogenic composition or vaccine formulation may include the aluminum-based adjuvant according to an aspect, and thereby, by improving the antigen content in the vaccine formulation and the adsorption rate to the antigen, the induction of the immune response may be enhanced compared to existing vaccine formulations.

Another aspect provides a kit including the immunogenic composition or a prefilled syringe filled with the immunogenic composition.

The immunogenic composition may be provided in the form of a kit including a container for administration in liquid form and/or instruction manual. As the container, a prefilled syringe, vial, syringe, single-use needle, microneedle patch, ampule, and dosing cartridge may be applied, and preferably a prefilled syringe, but is not limited thereto.

The kit may be packaged in a unit dosage or multiple dosage form (for example, 2 doses, 4 doses, or more doses). In the case of a multiple dosage form, it may be provided in the form of a vial, and a suitable multiple dosage form may include 2 doses to 10 doses per container (for example, 0.1 mL to 2 mL per dose), but is not limited thereto.

In an embodiment, when the immunogenic composition is provided in a liquid formulation, the kit may be provided in the form of a prefilled syringe, and a sterile needle may be mounted on the inlet of the prefilled syringe to be administered to a subject.

In an embodiment, when the immunogenic composition is provided in a lyophilized formulation and the immunogenic composition is to be reconstituted, the kit may be provided in the form of two or more vials, two or more pre-filled syringes, or one or more of each of these containers. In this case, the contents of the syringe may be used to reconstitute the contents of the vial prior to injection, or the contents of the vial may be used to reconstitute the contents of the syringe prior to injection.

Advantageous Effects

The aluminum-based adjuvant produced by the method according to an aspect may enhance the immune response induced by vaccination by increasing the loading of the target antigen in the vaccine formulation and the adsorption rate of the target antigen to the adjuvant.

Therefore, the method according to an aspect and the aluminum-based adjuvant produced by the method may be utilized to improve the safety and efficacy of existing vaccine formulations including polysaccharide vaccines and conjugate vaccines.

Description of Drawings

FIG. 1 shows the results of quantifying the geometric mean titer (GMT) of IgG antibodies against pneumococcal serotype 1 polysaccharide-protein conjugate antigen at 28 days after administration of a vaccine formulation according to an embodiment to an animal model.

FIG. 2 shows the results of quantifying the geometric mean titer (GMT) of IgG antibodies against pneumococcal serotype 3 polysaccharide-protein conjugate antigen at 28 days after administration of a vaccine formulation according to an embodiment to an animal model.

FIG. 3 shows the results of quantifying the geometric mean titer (GMT) of IgG antibodies against pneumococcal serotype 4 polysaccharide-protein conjugate antigen at 28 days after administration of a vaccine formulation according to an embodiment to an animal model.

FIG. 4 shows the results of quantifying the geometric mean titer (GMT) of IgG antibodies against pneumococcal serotype 5 polysaccharide-protein conjugate antigen at 28 days after administration of a vaccine formulation according to an embodiment to an animal model.

FIG. 5 shows the results of quantifying the geometric mean titer (GMT) of IgG antibodies against pneumococcal serotype 6A polysaccharide-protein conjugate antigen at 28 days after administration of a vaccine formulation according to an embodiment to an animal model.

FIG. 6 shows the results of quantifying the geometric mean titer (GMT) of IgG antibodies against pneumococcal serotype 6B polysaccharide-protein conjugate antigen at 28 days after administration of a vaccine formulation according to an embodiment to an animal model.

FIG. 7 shows the result of quantifying the geometric mean titer (GMT) of IgG antibodies against pneumococcal serotype 7F polysaccharide-protein conjugate antigen at 28 days after administration of a vaccine formulation according to an embodiment to an animal model.

FIG. 8 shows the result of quantifying the geometric mean titer (GMT) of IgG antibodies against pneumococcal serotype 8 polysaccharide-protein conjugate antigen at 28 days after administration of a vaccine formulation according to an embodiment to an animal model.

FIG. 9 shows the results of quantifying the geometric mean titer (GMT) of IgG antibodies against pneumococcal serotype 10A polysaccharide-protein conjugate antigen at 28 days after administration of a vaccine formulation according to an embodiment to an animal model.

FIG. 10. shows the results of quantifying the geometric mean titer (GMT) of IgG antibodies against pneumococcal serotype 11A polysaccharide-protein conjugate antigen at 28 days after administration of a vaccine formulation according to an embodiment to an animal model.

FIG. 11 shows the results of quantifying the geometric mean titer (GMT) of IgG antibodies against pneumococcal serotype 12F polysaccharide-protein conjugate antigen at 28 days after administration of a vaccine formulation according to an embodiment to an animal model.

FIG. 12 shows the results of quantifying the geometric mean titer (GMT) of IgG antibodies against pneumococcal serotype 14 polysaccharide-protein conjugate antigen at 28 days after administration of a vaccine formulation according to an embodiment to an animal model.

FIG. 13 shows the results of quantifying the geometric mean titer (GMT) of IgG antibodies against pneumococcal serotype 15B polysaccharide-protein conjugate antigen at 28 days after administration of a vaccine formulation according to an embodiment to an animal model.

FIG. 14 shows the results of quantifying the geometric mean titer (GMT) of IgG antibodies against pneumococcal serotype 18C polysaccharide-protein conjugate antigen at 28 days after administration of a vaccine formulation according to an embodiment to an animal model.

FIG. 15 shows the results of quantifying the geometric mean titer (GMT) of IgG antibodies against pneumococcal serotype 19A polysaccharide-protein conjugate antigen at 28 days after administration of a vaccine formulation according to an embodiment to an animal model.

FIG. 16 shows the results of quantifying the geometric mean titer (GMT) of IgG antibodies against pneumococcal serotype 19F polysaccharide-protein conjugate antigen at 28 days after administration of a vaccine formulation according to an embodiment to an animal model.

FIG. 17 shows the results of quantifying the geometric mean titer (GMT) of IgG antibodies against pneumococcal serotype 22F polysaccharide-protein conjugate antigen at 28 days after administration of a vaccine formulation according to an embodiment to an animal model.

FIG. 18 shows the results of quantifying the geometric mean titer (GMT) of IgG antibodies against pneumococcal serotype 23F polysaccharide-protein conjugate antigen at 28 days after administration of a vaccine formulation according to an embodiment to an animal model.

FIG. 19 shows the results of quantifying the geometric mean titer (GMT) of IgG antibodies against pneumococcal serotype 33F polysaccharide-protein conjugate antigen at 28 days after administration of a vaccine formulation according to an embodiment to an animal model.

Mode for Invention

Hereinafter, preferred examples are presented to assist in the understanding of the present disclosure. However, the following embodiments are provided only to facilitate understanding of the present disclosure, and the content of the present disclosure is not limited by the following embodiments.

[Example]

**Example 1. Autoclave treatment process for manufacturing an adjuvant**

In this example, 1.0 L to 1.6 L of a solution (suspension) containing aluminum phosphate particles was added to a 2 L reaction vessel (glass bottle), and then an autoclave treatment process was performed according to the following conditions, in which the temperature was sequentially increased a total of three times. The autoclave treatment conditions are as shown in Table 1 below. Here, the aluminum phosphate was Adju-Phos® adjuvant (vac-phos-250, Croda, which was sterilized at least 121 °C for at least 30 minutes by moist heat). Thereafter, the autoclaved solution was cooled down for 30 minutes to obtain the aluminum-based adjuvant according to an embodiment.

At this time, in order to stably reach each temperature condition of the autoclave, a pre-vacuum process was performed prior to the first process (0 bar, counter 1 time). By performing the pre-vacuum process, steam that could be discharged through the bypass of the sterilizer was effectively blocked, and through this, it was confirmed through experiments that the autoclave was performed in a stable cycle.

[Table 1]

|  | process | Temperature condition | Time |
|---|---|---|---|
| **Example 1** | 1st process | 102±0.1 °C | 3 min |
|  | 2nd process | 105±0.1 °C | 25 min |
|  | 3rd process | 118±0.1 °C | 240 min |

Meanwhile, during this process, the pressure inside the sterilizer chamber gradually increased from 0 bar in the range of the 1st process to the 2nd process, and reached about 1.3 bar when the temperature increase range of the 3rd process was reached. It was confirmed that this pressure was maintained at about 1.2 to 1.3 bar for a certain period of time, then decreased to about 0.8 bar just before the cooling process, and finally, after the cooling process, the pressure inside the chamber decreased to about 0.2 bar.

[Comparative Examples]

Comparative Example 1. Preparation of aluminum-based adjuvants without autoclave treatment process

An aluminum-based adjuvant was prepared using the same amount of solution containing aluminum phosphate particles as in Example 1 but without applying the autoclave treatment process of Example 1.

**Comparative Examples 2 and 3. Preparation of aluminum-based adjuvants by autoclave treatment process under modified conditions**

The aluminum-based adjuvant was prepared by performing the autoclave treatment process under the following conditions, in which the temperature and treatment time were modified compared to Example 1, although the same method as in Example 1 was employed. Specifically, in these conditions, the initial autoclave temperature (1st cycle and/or 2nd cycle) was set higher than in Example 1, and the treatment time was set longer.

[Table 2]

| | process | Temperature condition | Time |
|---|---|---|---|
| **Comparative Example 2** | 1st process | 116±0.1 °C | 30 min |
| | 2nd process | 122±0.1 °C | 20 min |
| | 3rd process | 123±0.1 °C | 60 min |

[Table 3]

| | process | Temperature condition | Time |
|---|---|---|---|
| **Comparative Example 3** | 1st process | 116±0.1 °C | 40 min |
| | 2nd process | 122±0.1 °C | 30 min |
| | 3rd process | 123±0.1 °C | 120 min |

**[Experimental Example]**

**Experimental Example 1. Evaluation of pH and particle size of aluminum-based adjuvant.**

In this experimental example, it was intended to confirm the effect of the autoclave treatment process of Example 1 on the physicochemical properties of the aluminum-based adjuvant. Specifically, aluminum phosphate adjuvant samples of Example 1 and aluminum phosphate adjuvant samples not subjected to the autoclave treatment process were prepared, and then 0.85 % NaCl solution was added to each to appropriately dilute them. Thereafter, the pH and Z-average particle size of these adjuvant samples were measured.

Table 4 shows the results of measuring the pH and particle size of the aluminum-based adjuvant.

[Table 4]

| | Example 1 | Comparative Example 1 |
|---|---|---|
| pH | 5.9 | 5.97 |
| Z-Average (nm) | 3048 | 3289 |

As a result, as shown in Table 4 above, in the case of undergoing the autoclave treatment process according to the present example, similar levels of pH and Z-average values to those of the comparative example that did not undergo the autoclave treatment process were observed. That is, despite the autoclave treatment process according to an embodiment, it was confirmed that the physicochemical properties of the aluminum-based adjuvant itself were maintained.

**Experimental Example 2. Evaluation of antigen content in vaccines containing adjuvant subjected to autoclave treatment process**

In this experimental example, it was intended to confirm the effect of the aluminum-based adjuvant subjected to the autoclave treatment process of Example 1 on the antigen content in the vaccine. Specifically, standard antigens and antibodies were prepared for each pneumococcal serotype polysaccharide (4, 5, 6A, 6B, 8, 9V, 10A, 11A, 14, 15B, 18C, 19F, and 22F) used as antigens in the vaccine. The standard stock solution was prepared by slowly mixing at room temperature for at least 2 hours to reach a content level of 6.6 $\mu$g/mL for each serotype polysaccharide-protein conjugate antigen. Thereafter, using the standard stock solution, standard solutions were prepared in which the content of each serotype polysaccharide-protein conjugate antigen was at the level of 3.1 $\mu$g/mL to 11.4 $\mu$g/mL, and standard solutions including the aluminum-based adjuvant subjected to the autoclave treatment process of Example 1 and standard solutions including the aluminum-based adjuvant not subjected to the autoclave treatment process were prepared. Thereafter, a neutralization reaction was induced using a 1.1 N NaOH solution and a 0.365 M citric acid solution for the vaccine formulation, that is, the standard solution. Subsequently, using the nephelometer method, which measures the degree of light scattering through antigen-antibody complexes, the level of antigen-antibody complexes generated for each serotype polysaccharide-protein conjugate antigen was evaluated according to the passage of time (0 month, 4 months, and 6 months), and through this, the antigen content was quantified. Specifically, a standard curve of the rate response of the standard solution was obtained by using the concentration ($\mu$g/mL) of the standard solution as the x-axis value and the mean rate response value of the standard solution as the y-axis value. Thereafter, the slope and y-intercept values from the standard curve were applied to the following equation to quantify the antigen content level for each serotype antigen.

Antigen content level = (Response value for the antigen - y-intercept / slope of the standard curve) * dilution factor [Equation 1]

Table 5 shows the results of quantifying the level of antigen content included in the vaccine formulation by serotype.

[Table 5]

| Serotype | Example 1 ($\mu$g/L) | Comparative Example 1 ($\mu$g/mL) |
|---|---|---|
| 4 | 8.8 | 8.7 |
| 5 | 4.0 | 4.0 |
| 6A | 4.2 | 4.2 |
| 6B | 7.6 | 7.6 |
| 8 | 4.1 | 3.9 |
| 9V | 9.2 | 7.5 |
| 10A | 4.8 | 4.6 |
| 11A | 4.5 | 4.3 |
| 14 | 4.5 | 4.5 |
| 15B | 4.2 | 4.1 |
| 18C | 4.6 | 4.5 |
| 19F | 9.6 | 9.3 |
| 22F | 4.6 | 4.6 |

As a result, as shown in Table 5 above, it was confirmed that when using the adjuvant subjected to the autoclave treatment process according to Example 1, the antigen content in the vaccine formulation was maintained or increased. In particular, considering that in vaccine formulations containing antigens derived from multiple serotypes, the final concentration of the antigen for a single serotype, for example, the pneumococcal serotype polysaccharide-protein conjugate antigen, is typically set at a low concentration of about 2.2 $\mu$g/dose, the effect of increasing the antigen content can be considered remarkable.

**Experimental Example 3. Evaluation of adsorption rate in vaccines containing adjuvant subjected to the autoclave treatment process**

In this experimental example, it was intended to confirm the effect of the aluminum-based adjuvant subjected to the autoclave treatment process of Example 1 on the adsorption rate of antigen in the vaccine. Specifically, standard antigens and antibodies were prepared for each pneumococcal serotype polysaccharide-protein conjugate antigen (3, 4, 6A, 7F, 8, 9N, 9V, 10A, 11A, 12F, 14, 18C, 19A, 19F, 22F, 23F, and 33F) used as antigens in the vaccine. Thereafter, the level of antigen-antibody complexes generated for each serotype polysaccharide-protein conjugate antigen in the vaccine formulation prepared in the same manner as in Experimental Example 3 was measured, and the antigen content was quantified. Thereafter, for the vaccine formulation, the supernatant containing the antigen not adsorbed to the adjuvant was removed using a centrifuge (8,000 rcf, 4 °C, 10 minutes), and the precipitate containing the adjuvant and the adsorbed antigen was resuspended using a vehicle. Thereafter, the antigen content in the resuspended vaccine formulation was quantified in the same manner as described above. Then, the ratio (%) of the antigen content in the resuspended vaccine formulation to the antigen content in the vaccine formulation was calculated to determine the adsorption rate for each serotype polysaccharide-protein conjugate antigen.

Table 6 shows the results of quantifying the adsorption rate of the antigen to the adjuvant in the vaccine formulation by serotype.

[Table 6]

| Serotype | Example 1 (%) | Comparative Example 1 (%) |
|----------|---------------|---------------------------|
| 3 | 39 | 37 |
| 4 | 51 | 46 |
| 6A | 52 | 45 |
| 7F | 42 | 33 |
| 8 | 37 | 36 |
| 9N | 37 | 31 |
| 9V | 54 | 52 |
| 10A | 56 | 50 |
| 11A | 60 | 49 |
| 12F | 13 | 8 |
| 14 | 44 | 40 |
| 18C | 39 | 36 |
| 19A | 55 | 51 |
| 19F | 59 | 57 |
| 22F | 11 | 6 |
| 23F | 31 | 30 |
| 33F | 60 | 60 |

As a result, as shown in Table 6 above, it was confirmed that when using the adjuvant subjected to the autoclave treatment process according to Example 1, the adsorption rate of the antigen in the vaccine formulation was generally increased. Considering that, as described above, the final concentration of the pneumococcal serotype polysaccharide-protein conjugate antigen in the vaccine formulation is typically set at a low concentration of about 2.2 $\mu$g/dose, the effect of increasing the antigen adsorption rate may be considered remarkable.

**Experimental Example 4. Comparison of efficacy according to autoclave treatment conditions**

In this experimental example, the efficacy was compared between the aluminum-based adjuvant subjected to the autoclave treatment process of Example 1 and the aluminum adjuvant prepared under modified autoclave treatment conditions (Comparative Examples 2 and 3).

**4-1. Antigen content evaluation**

In the same manner as in Experimental Example 2, vaccine formulations for each pneumococcal serotype polysaccharide-protein conjugate antigen and antibodies for each antigen were prepared, and the antigen content levels included in the vaccine formulation were compared.

Table 7 shows the results of quantifying and comparing the levels of antigen content by serotype of the polysaccharide-protein conjugate antigens included in the vaccine formulations of Example 1 and Comparative Example 2.

[Table 7]

| Serotype | Example 1 ($\mu$g/mL) | Comparative Example 2 ($\mu$g/mL) |
|---|---|---|
| 1 | 4.8 | 4.8 |
| 3 | 11.0 | 9.5 |
| 9N | 4.6 | 4.4 |
| 14 | 4.5 | 4.3 |
| 18C | 4.6 | 4.2 |
| 19A | 9.6 | 9.5 |
| 19F | 9.6 | 8.7 |
| 33F | 4.3 | 4.3 |

As a result, as shown in Table 7 above, it was confirmed that when using the adjuvant subjected to the autoclave treatment process according to Example 1, the antigen content in the vaccine formulation was generally increased. That is, the autoclave treatment process according to Example 1 reduced the level of antigen loss that could be caused during the manufacturing process, confirming that it may contribute to providing an adjuvant/vaccine formulation with improved efficacy.

4-2. Evaluation of adsorption rate

In the same manner as in Experimental Example 3, vaccine formulations for each pneumococcal serotype polysaccharide-protein conjugate antigen and antibodies for each antigen were prepared, and the antigen adsorption rates by serotype polysaccharide-protein conjugate antigen in the vaccine formulation were compared.
Table 8 shows the results of quantifying and comparing the adsorption rate of the antigen to the adjuvant in the vaccine formulation of Example 1 and Comparative Example 2 by serotype, and Table 9 shows the results of quantifying and comparing the adsorption rate of the antigen to the adjuvant in the vaccine formulation of Example 1 and Comparative Example 3 by serotype.

[Table 8]

| Serotype | Example 1 (%) | Comparative Example 2 (%) |
|---|---|---|
| 7F | 42 | 32 |
| 10A | 56 | **55** |
| 11A | 60 | 39 |
| 23F | 31 | 17 |

[Table 9]

| Serotype | Example 1 (%) | Comparative Example 3 (%) |
|---|---|---|
| 4 | 59 | 56 |
| 6A | 48 | 48 |
| 7F | 46 | 45 |
| 9N | 41 | 41 |
| 9V | 52 | 52 |
| 15B | 11 | 11 |
| 18C | 43 | 43 |
| 19A | 63 | 60 |
| 22F | 2 | 0 |
| 23F | 38 | 36 |

As a result, as shown in Tables 8 and 9 above, it was confirmed that when using the adjuvant subjected to the autoclave treatment process according to Example 1, the antigen adsorption rate in the vaccine formulation was generally maintained or increased. Moreover, in the cases of Comparative Examples 2 and 3, which showed adsorption rates similar to that of Example 1, aggregation of aluminum salt particles was observed, confirming that the uniformity of the vaccine formulation was reduced. That is, the autoclave treatment process according to Example 1 not only achieved an enhanced immuno-enhancing effect derived from the improved adsorption rate but also showed that it may be applied to commercial scale manufacturing processes by improving the stability of the formulation.

**Experimental Example 5. Evaluation of the immunogenicity of the vaccine formulation using an animal model**

In this experimental example, it was intended to confirm the effect of the aluminum-based adjuvant subjected to the autoclave treatment process of Example 1, which exhibited excellent antigen content and antigen adsorption rate as described above, on the immunogenicity of the vaccine through an animal model. For this purpose, after administering to an animal model (rabbit) a vaccine formulation containing the adjuvant subjected to the autoclave treatment process (Example 1) and a vaccine formulation containing the adjuvant not subjected to the autoclave treatment process (Comparative Example 1), the IgG antibody titer generated by the vaccine formulation was measured using PnPS ELISA (Pneumococcal Polysaccharide Enzyme-Linked Immunosorbent Assay) using the serum of the vaccinated animal model as the sample. Specifically, the surface of a 96-well plate was coated with an antigen at 5 or 10 $\mu$g/mL, and then incubated at 37 °C for 5 hours. After washing the incubated plate, it was blocked with a BSA solution at 37 °C for 1 hour. Thereafter, the plate was washed and then a serially diluted serum dilution was dispensed into the plate. A secondary antibody, goat anti-rabbit IgG-alkaline phosphatase conjugate, was diluted and added to the plate and reacted at room temperature for 2 hours. After washing the plate, 1 mg/mL p-nitrophenylamine buffer was added, and then reacted again at room temperature for 2 hours. Thereafter, 3 M NaOH was added to each well to stop the reaction, and the absorbance at 405 nm and 690 nm was measured to calculate the geometric mean titer (GMT) of the IgG antibody.

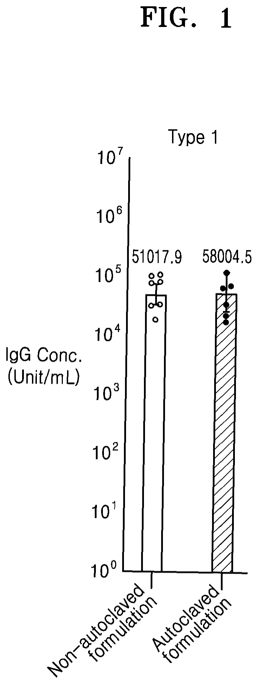

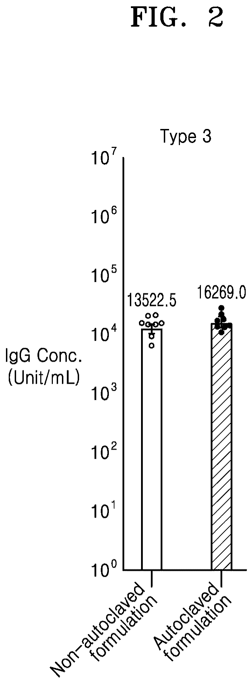

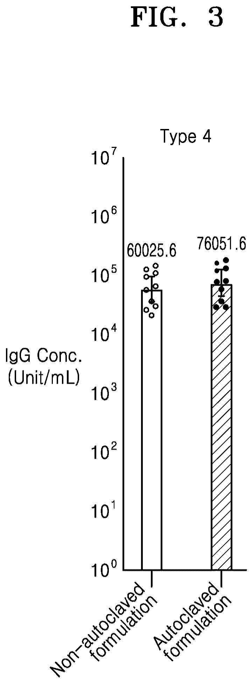

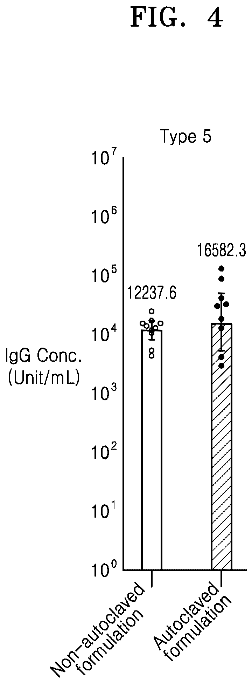

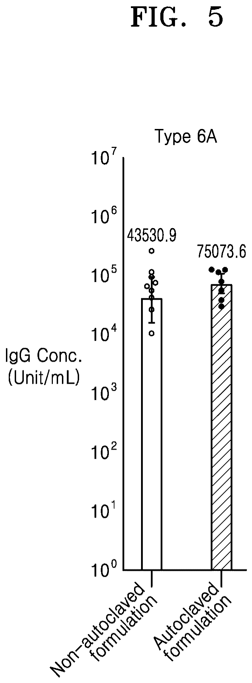

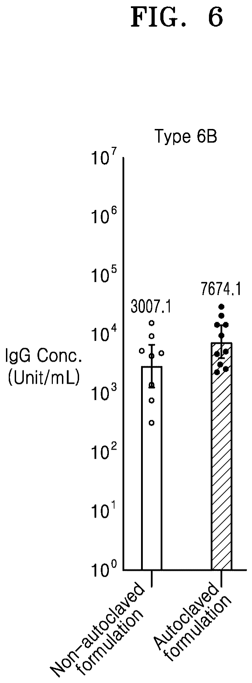

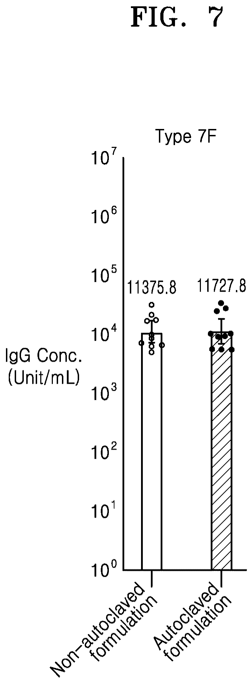

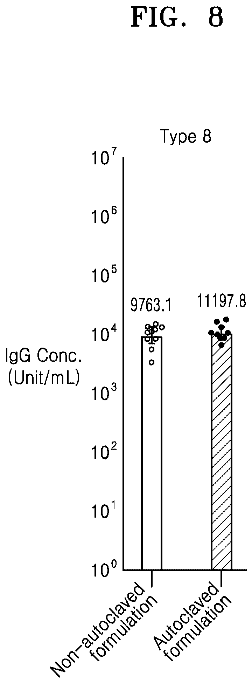

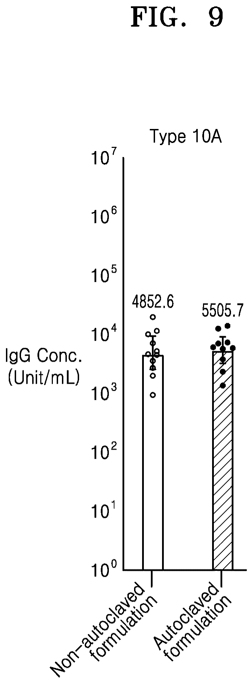

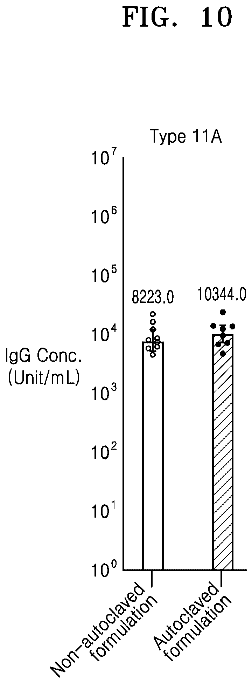

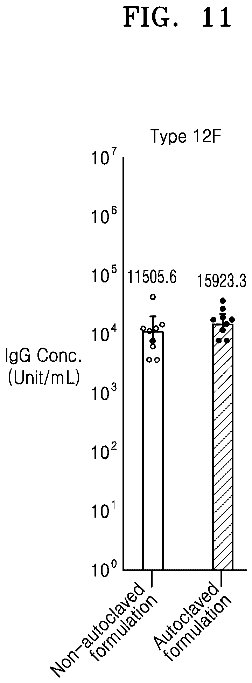

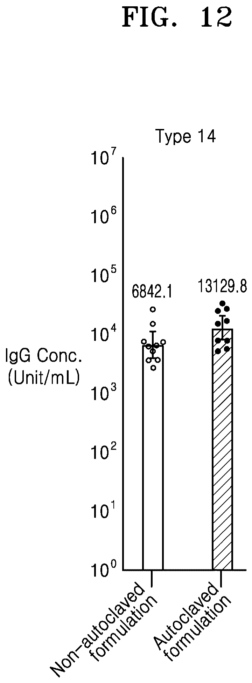

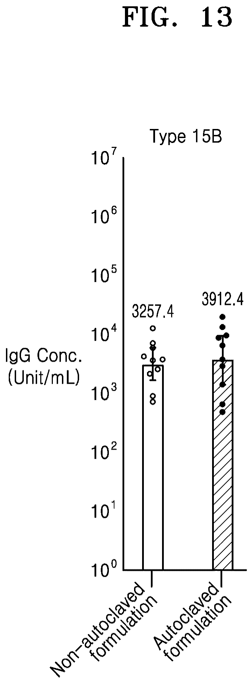

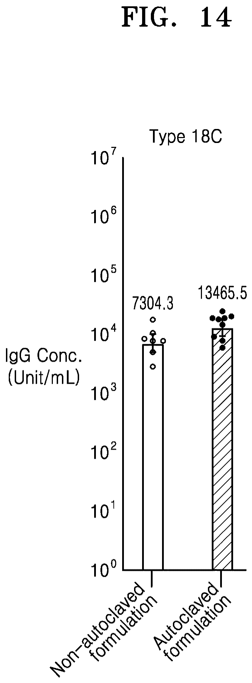

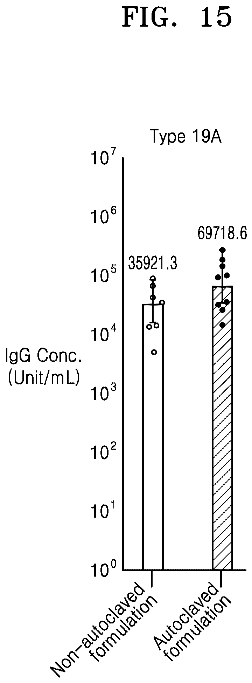

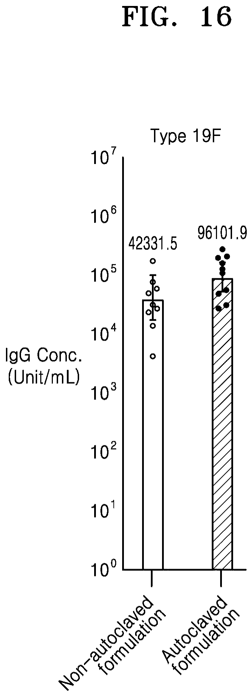

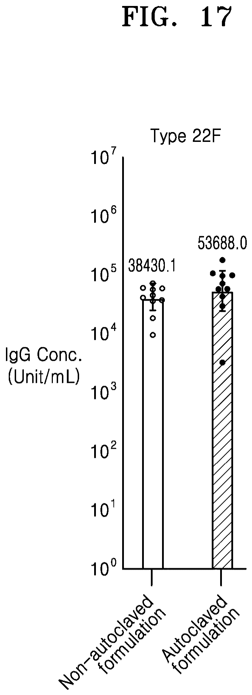

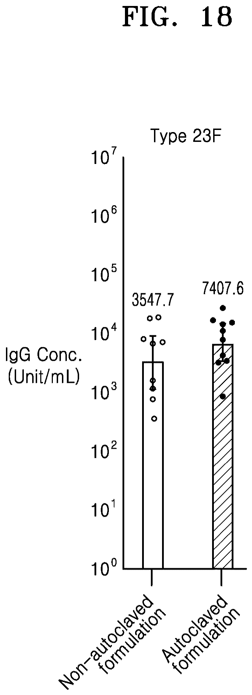

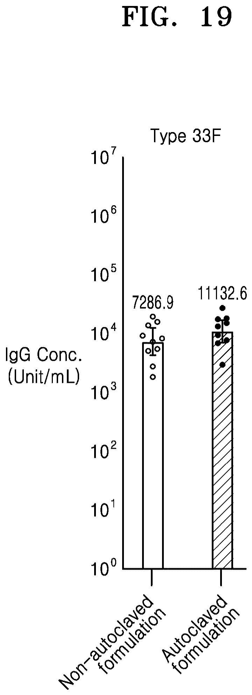

As a result, as shown in FIGS. 1 to 19, it was confirmed that the induction of the immune response by vaccination was enhanced when using the adjuvant subjected to the autoclave treatment process according to Example 1.

The above description of the present disclosure is for illustrative purposes, and it will be understood by those of ordinary skill in the art to which the present disclosure pertains that various modifications may be easily made without changing the technical spirit or essential features of the present disclosure. Therefore, it should be understood that the embodiments described above are illustrative in all respects and not restrictive.

**Claims**

1. A method of preparing an aluminum-based adjuvant, comprising a plurality of processes of autoclave treatment in which temperature is sequentially increased in a solution containing aluminum salt particles, wherein the processes comprise:

   a first process of applying steam at 101 °C to 103 °C for 2 minutes to 4 minutes to the solution containing the aluminum salt particles;
   a second process of applying steam at 104 °C to 106 °C for 20 minutes to 30 minutes to the solution having undergone the first process; and
   a third process of applying steam at 110 °C to 126 °C for 220 minutes to 260 minutes to the solution having undergone the second process.

2. The method of claim 1, wherein the method further comprises a process of cooling down after the autoclave treatment.

3. The method of claim 1, wherein the aluminum-based adjuvant has one or more of the following effects:

   (a) enhanced loading of a target antigen;
   (b) enhanced adsorption rate with the target antigen; and
   (c) enhanced immunogenicity of the target antigen.

4. The method of claim 1, wherein the solution containing the aluminum salt particles is any one of an aluminum phosphate solution, an aluminum sulfate solution, an aluminum hydroxide solution, a potassium aluminum sulfate solution, and an aluminum hydroxyphosphate solution.

5. The method of claim 3, wherein the target antigen is one or more of a protein antigen, a recombinant protein antigen, a polysaccharide antigen, and a polysaccharide-protein conjugate antigen.

6. The method of claim 5, wherein the target antigen is at least one or more of the following pneumococcal serotype polysaccharide antigens: 1, 2, 3, 4, 5, 6A, 6B, 6C, 5D, 7A, 7C, 7F, 8, 9N, 9V, 10A, 10F, 11A, 11B, 12F, 13, 14, 15A, 15B, 15C, 15F, 16F, 17F, 18C, 19A, 19B, 19F, 20, 20A, 21, 22F, 23A, 23B, 23F, 24F, 25A, 31, 33F, 35A, 35B, 35D, 35F, 38, and 39.

7. An aluminum-based adjuvant prepared by the method of any one of claims 1 to 6.

8. An immunogenic composition comprising: a polysaccharide-protein conjugate comprising a pneumococcal serotype polysaccharide antigen; and the aluminum-based adjuvant of claim 7.

9. The immunogenic composition of claim 8, wherein the pneumococcal serotype polysaccharide antigen is at least one or more of 1, 2, 3, 4, 5, 6A, 6B, 6C, 5D, 7A, 7C, 7F, 8, 9N, 9V, 10A, 10F, 11A, 11B, 12F, 13, 14, 15A, 15B, 15C, 15F, 16F, 17F, 18C, 19A, 19B, 19F, 20, 20A, 21, 22F, 23A, 23B, 23F, 24F, 25A, 31, 33F, 35A, 35B, 35D, 35F, 38, and 39.

10. The immunogenic composition of claim 8, wherein the aluminum-based adjuvant is any one of an aluminum phosphate adjuvant, an aluminum sulfate adjuvant, an aluminum hydroxide adjuvant, a potassium aluminum sulfate adjuvant, and an aluminum hydroxyphosphate adjuvant.

11. The immunogenic composition of claim 8, wherein the immunogenic composition is formulated to include sodium chloride, succinate, and any one of polysorbate 80 or polysorbate 20 as a surfactant.

12. A kit comprising the immunogenic composition of claim 8.

13. A prefilled syringe filled with the immunogenic composition of claim 8.

# FIG. 1

# FIG. 2

# FIG. 3

# FIG. 4

# FIG. 5

# FIG. 6

# FIG. 7

# FIG. 8

Type 8

IgG Conc. (Unit/mL)

9763.1 — Non-autoclaved formulation

11197.8 — Autoclaved formulation

# FIG. 9

# FIG. 10

Type 11A

# FIG. 11

# FIG. 12

FIG. 13

# FIG. 14

Type 18C

IgG Conc. (Unit/mL)

7304.3

13465.5

Non-autoclaved formulation

Autoclaved formulation

# FIG. 15

# FIG. 16

Type 19F

# FIG. 17

# FIG. 18

# FIG. 19

## INTERNATIONAL SEARCH REPORT

| International application No. |
|---|
| **PCT/KR2024/004079** |

### A. CLASSIFICATION OF SUBJECT MATTER

**A61K 39/39**(2006.01)i; **A61K 39/09**(2006.01)i; **A61P 31/04**(2006.01)i; **A61K 39/00**(2006.01)i

According to International Patent Classification (IPC) or to both national classification and IPC

### B. FIELDS SEARCHED

Minimum documentation searched (classification system followed by classification symbols)

A61K 39/39(2006.01); A61K 31/7088(2006.01); A61K 31/711(2006.01); A61K 39/00(2006.01): A61K 39/08(2006.01); A61K 39/385(2006.01); A61K 47/48(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 알루미늄(aluminum), 오토클레이브(autoclave), 애주번트(adjuvant), 항원(antigen), 폐렴구균(Streptococcus pneumoniae), 다당류-단백질 접합체(Carbohydrate-protein conjugate)

### C. DOCUMENTS CONSIDERED TO BE RELEVANT

| Category* | Citation of document, with indication, where appropriate, of the relevant passages | Relevant to claim No. |
|---|---|---|
| A | KR 10-2014-0100530 A (MERCK SHARP & DOHME CORP.) 14 August 2014 (2014-08-14) <br> See entire document. | 1-13 |
| A | KR 10-2019-0055063 A (SANOFI PASTEUR, INC. et al.) 22 May 2019 (2019-05-22) <br> See entire document. | 1-13 |
| A | KR 10-2018-0129903 A (DYNAVAX TECHNOLOGIES CORPORATION) 05 December 2018 (2018-12-05) <br> See entire document. | 1-13 |
| A | WO 2012-136823 A1 (GLAXOSMITHKLINE BIOLOGICALS S.A. et al.) 11 October 2012 (2012-10-11) <br> See entire document. | 1-13 |
| A | KR 10-2022-0004085 A (XIAMEN INNOVAX BIOTECH CO., LTD. et al.) 11 January 2022 (2022-01-11) <br> See entire document. | 1-13 |

☐ Further documents are listed in the continuation of Box C.    ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **26 June 2024** | **01 July 2024** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/004079**

| Patent document cited in search report | | | Publication date (day/month/year) | Patent family member(s) | | | Publication date (day/month/year) |
|---|---|---|---|---|---|---|---|
| KR | 10-2014-0100530 | A | 14 August 2014 | CN | 109862908 | A | 07 June 2019 |
| | | | | CN | 109862908 | B | 02 May 2023 |
| | | | | EP | 3493837 | A1 | 12 June 2019 |
| | | | | EP | 3493837 | A4 | 08 April 2020 |
| | | | | EP | 3493837 | B1 | 31 August 2022 |
| | | | | JP | 2019-526621 | A | 19 September 2019 |
| | | | | JP | 7001687 | B2 | 04 February 2022 |
| | | | | KR | 10-2437120 | B1 | 25 August 2022 |
| | | | | US | 11241489 | B2 | 08 February 2022 |
| | | | | US | 2020-0237889 | A1 | 30 July 2020 |
| | | | | WO | 2018-027126 | A1 | 08 February 2018 |
| KR | 10-2019-0055063 | A | 22 May 2019 | EP | 2782594 | A1 | 01 October 2014 |
| | | | | EP | 2782594 | A4 | 29 July 2015 |
| | | | | EP | 2782594 | B1 | 17 April 2019 |
| | | | | KR | 10-2032002 | B1 | 14 October 2019 |
| | | | | US | 2014-0314653 | A1 | 23 October 2014 |
| | | | | US | 9573811 | B2 | 21 February 2017 |
| | | | | WO | 2013-078102 | A1 | 30 May 2013 |
| KR | 10-2018-0129903 | A | 05 December 2018 | CN | 109475612 | A | 15 March 2019 |
| | | | | EP | 3442568 | A1 | 20 February 2019 |
| | | | | EP | 3442568 | A4 | 18 December 2019 |
| | | | | JP | 2019-515900 | A | 13 June 2019 |
| | | | | US | 2017-0326232 | A1 | 16 November 2017 |
| | | | | WO | 2017-181128 | A1 | 19 October 2017 |
| WO | 2012-136823 | A1 | 11 October 2012 | CN | 103458925 | A | 18 December 2013 |
| | | | | CN | 200369885 | Y | 22 March 2000 |
| | | | | CN | 201016869 | Y | 06 February 2008 |
| | | | | EP | 2694104 | A1 | 12 February 2014 |
| | | | | JP | 2014-510129 | A | 24 April 2014 |
| | | | | US | 2014-0030342 | A1 | 30 January 2014 |
| KR | 10-2022-0004085 | A | 11 January 2022 | CN | 111803627 | A | 23 October 2020 |
| | | | | CN | 111803627 | B | 03 January 2023 |
| | | | | EP | 3954386 | A1 | 16 February 2022 |
| | | | | EP | 3954386 | A4 | 06 July 2022 |
| | | | | JP | 2022-527606 | A | 02 June 2022 |
| | | | | US | 2022-0193231 | A1 | 23 June 2022 |
| | | | | WO | 2020-207472 | A1 | 15 October 2020 |

Form PCT/ISA/210 (patent family annex) (July 2022)

**EP 4 691 486 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230043089

- KR 101540920